(19) Europäisches Patentamt
European Patent Office
Office européen des brevets

(11) Publication number: 0 518 440 A1

(12) EUROPEAN PATENT APPLICATION

(21) Application number: 92201702.5

(51) Int. Cl.5: C07C 37/56, C07C 39/04

(22) Date of filing: 11.06.92

(30) Priority: 14.06.91 BE 9100583

(43) Date of publication of application:
16.12.92 Bulletin 92/51

(84) Designated Contracting States:
AT BE CH DE DK ES FR GB GR IT LI NL PT SE

(71) Applicant: DSM N.V.
Het Overloon 1
NL-6411 TE Heerlen(NL)

(72) Inventor: Buijs, Wim
Wolfhagen 145
NL-6365 BM Schinnen(NL)

# (54) Process for the preparation of a phenol.

(57) Process for the preparation of a phenol by an oxidative decarboxylation in the liquid phase of a corresponding arylcarboxylic acid in the presence of a Cu(I)-containing catalyst, characterized in that the following process steps are performed:

a) oxidation of the catalyst at a temperature of 120-260 °C, with the temperature, retention time, oxidation agent, phenol concentration and Cu(I) concentration being chosen so that virtually no tar is formed,

b) reaction of the oxidized catalyst of step a) and formation of phenol in the absence of oxygen, in the presence of water, at a temperature of 210-270 °C,

c) separation of phenol and recycling of the (reduced) catalyst to step a),

4-10 wt.% Cu being present in the reaction mixture, in step a) such a quantity of Cu(I) salt being converted to Cu(II) salt that the quantity of Cu(II) salt is higher than its solubility product so that in step a) a slurry is formed, nucleation agents for crystallization being added in step a), step b) being carried out in a cascade of at least two reactors, and the reduction of the catalyst being continued so far that a homogeneous solution is obtained.

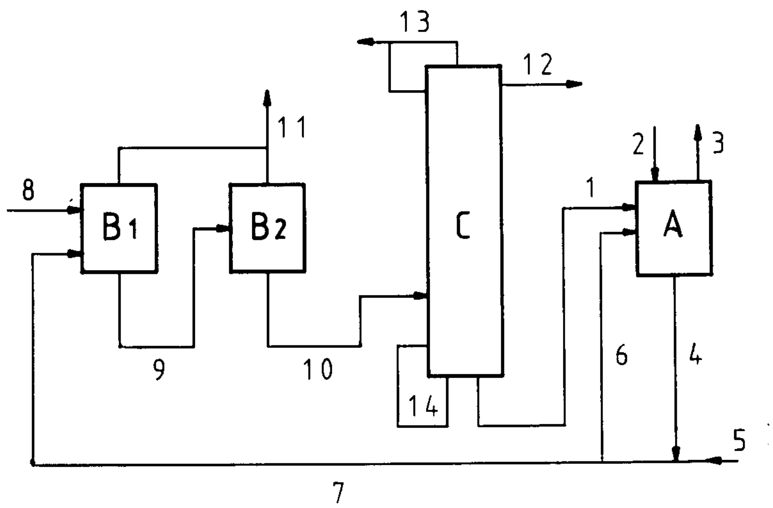


EP 0 518 440 A1

The invention relates to a process for the preparation of a phenol by an oxidative decarboxylation in the liquid phase of a corresponding arylcarboxylic acid in the presence of a Cu(I)-containing catalyst.

The preparation of a phenol by an oxidative decarboxylation has long been known. NL-B-90.684 already discloses such a process, wherein the oxidation, the decarboxylation as well as the reduction are effected in a single process step, at a temperature of at least 200 ° C, preferably 230-250 ° C.

In relation with this process a number of patent publications have appeared over the years, aiming to suppress the principal drawback of said process, which is the formation of a considerable number of by-products, mainly in the form of tar.

In NL-A-70.00685 a two-step process for the preparation of a phenol from a benzene monocarboxylic acid is described. First, the oxidation and the decarboxylation are carried out simultaneously at a temperature of 230-240 ° C. Next, hydrolysis of the resulting corresponding phenylbenzoate is effected in the presence of oxygen at a temperature of about 200 ° C.

NL-A-78.07199 describes a process for the preparation of a phenol on the basis of a three-step process. First, an oxidation is effected in the absence of water, at a temperature of preferably 120-170 ° C; then a decarboxylation in the absence of oxygen and water, at a temperature preferably below 220 ° C, after which in a third step a hydrolysis of the resulting arylbenzoate takes place in the absence of oxygen, preferably at a temperature of about 220 ° C. According to the applicant for the patent in question, the presence of water in the decarboxylation should be avoided, which is achieved by addition of a dehydrating agent, by azeotropic distillation with an extra hydrocarbon added or by stripping with a dry inert gas.

However, all said processes have not enabled the formation of the phenol to be accomplished with a high yield under economically sound circumstances.

The process according to the invention provides a process for the preparation of a phenol from a corresponding arylcarboxylic acid with elimination of the drawbacks encountered in the above-mentioned processes. Thus a process is obtained which combines high preparation selectivities with an economically attractive embodiment. The process according to the invention is characterized in that the following process steps are effected:

a) oxidation of the catalyst at a temperature of 120-260 ° C, with the temperature, retention time, oxidation agent, phenol concentration and Cu(I) concentration being chosen so that virtually no tar is formed,

b) reaction of the oxidized catalyst of step a) and formation of phenol in the absence of oxygen, in the presence of water, at a temperature of 210-270 ° C,

c) separation of phenol and recycling of the (reduced) catalyst to step a), 4-10 wt.% Cu being present in the reaction mixture, in step a) such a quantity of Cu(I) salt being converted to Cu(II) salt that the quantity of Cu(II) salt is higher than its solubility product so that in step a) a slurry is formed, nucleation agents for crystallization being added in step a), and step b) being carried out in a cascade of at least two reactors, the reduction of the catalyst being continued so far that a homogeneous solution is obtained.

By performing the process in this way, a two-step process is obtained: first an oxidation, then a reduction and formation of the phenol.

In this way the number of process steps is reduced in comparison with the process described in NL-A-78.07199. In addition it appears that the process according to the invention gives a higher yield.

Here and below, arylcarboxylic acid is understood to be a compound having the following structure:

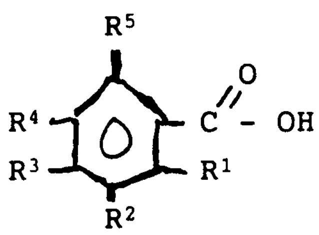

(I)

where $R^1$ through $R^5$ may be hydrogen (on the proviso that at least $R^1$ or $R^5$ is hydrogen) or organic groups, which have a so-called Hammett constant of between -1 and +2. A description of this Hammett or $\sigma$ value, which represents a measure of the influence of the group on the reactivity of the arylcarboxylic acid, can be found in J. March, Advanced Organic Chemistry 1989, pages 242-250; see in particular table 4 on page 244. The groups that can be used therefore are: $C_1$-$C_6$ alkyl, cylcoalkyl, aryl, arylalkyl, amino, halogen, nitro.

Salts, esters and anhydrides of (I) are also suitable, while the groups may also be connected with each other via a ring system, as is the case for instance in naphthalene carboxylic acid (substituted or not).

Multiple arylcarboxylic acids, such as trimellitic acid and pyromellitic acid, can also be used as starting material. Mixtures of the arylcarboxylic acids described above can also be used in the process according to the invention.

The invention relates particularly to a process for the conversion of unsubstituted benzoic acid ($R^1$ through $R^5$ = hydrogen) into the corresponding unsubstituted phenol.

The oxidation of the Cu-containing catalyst, involving conversion of Cu(I) arylcarboxylate into Cu(II) arylcarboxylate, is a first reaction step in the process. It brings about an increase in the degree of oxidation of the copper (from 1+ to 2+) as well as incorporation of an extra arylcarboxylate part into the Cu-containing catalyst. The process conditions for this need to be chosen such that the reaction can be carried out in the liquid phase (i.e. above the melting point of the arylcarboxylic acid to be converted) and that no significant tar formation can occur yet.

This can be achieved by several measures. For instance, the temperature can be kept below 200 °C, preferably between 150-190 °C, because then oxidation of copper takes place already, but no tar is formed yet.

It is also possible to apply a higher temperature (e.g. 191-260 °C, preferably 210-250 °C) and keep the contact time between the reaction mixture and oxygen short, for instance shorter than 20 minutes, in particular 3-10 minutes, with supply of such a quantity of oxygen that not all Cu(I) is oxidized to Cu(II). In particular, such a quantity of oxygen is supplied that at least 0.04 wt.% copper (relative to the reaction mass) remains present as Cu(I) salt. The effect of these measures is that tar formation - which affects the selectivity - is virtually absent.

The oxidation of Cu(I) to Cu(II) proceeds well particularly when carried out using an oxygen-containing gas. Air, whether or not enriched with oxygen or oxygen-depleted, can very well be used for this. An oxygen content of the gas of between 1-15% is very suitable. Such a gas can be passed through the Cu-containing liquid, for instance in a bubble-type washer. The pressure applied is not critical, but in general an elevated pressure will be chosen so as to accelerate the oxidation process. Pressures of 0.1-2.5 MPa are therefore suitable. If it is sought to prevent oxidation of all Cu(I) to Cu(II), the degree of oxidation can be determined by measuring the amount of oxygen supplied relative to the amount of Cu(I) salts added. When too much or too little oxygen threatens to be supplied, less oygen or extra oxygen can be supplied to the oxidation gas flow.

An alternative and very suitable method for bringing about the conversion of Cu(I) into Cu(II) is to use the effect of an electrochemical potential, in which case the abstraction of an electron enables the desired copper conversion to take place.

The applicant has found that for the conversion of Cu(I) to Cu(II) using an oxygen-containing gas (or other means by which Cu oxidation can be effected via an oxygen group, such as ozone or $N_2O$) preference is to be given to the use of a deficiency of oxygen relative to the amount of Cu in the catalyst. In this way it is ensured that not all of the Cu is in the Cu(II) form. In particular it is desirable to have 0.04 wt.% copper (relative to the reaction mass) remaining as Cu(I) salt. For electrochemical conversion of Cu(I) to Cu(II) such a measure was found to be unnecessary.

The amount of Cu-containing catalyst is to be chosen so that a good activity is obtained, but preferably it should not be so large as to give rise to the presence of a separate, solid catalyst phase throughout the process. The catalyst is dissolved as Cu(I) salt in the reaction mixture. The copper concentration (as metal) in the oxidation step therefore is 4-10 wt.%, more preferably 5-8 wt.% (all relative to the reaction mixture in the oxidation step). As the solubility of Cu(II) arylcarboxylate is lower than that of Cu(I) arylcarboxylate, the amount of Cu(II) salt comes to exceed the solubility product of that salt in that medium during the oxidation step. As a consequence, a precipitate of Cu(II) salt is formed. For a controlled slurry formation it is necessary to add nucleation agents, preferably in the form of a recirculation flow from a line leading to a reactor where a second step is effected or from the first reactor where the second step is effected. In this way, part of the slurry is recycled from step b to step a.

There may be advantage in using a catalyst containing a co-catalyst besides copper. This co-catalyst can be chosen in particular from groups V, VI, VII and VIII as well as from the group of the lanthanides and actinides of the Periodic System of the Elements. These components have an effect on the oxidative capacity of the Cu in the catalyst. Furthermore, promoters may be used, suitable materials being in particular (earth) alkaline metals, such as Mg or Li. These promoters in general enhance the solubility of the copper salts.

Preferably, these co-catalysts and/or promoters are used in a quantity of 0.5-10 wt.%.

The second step in the process according to the invention comprises a combination of a reduction and the formation of the phenol, in which process carbondioxide ($CO_2$) is released. This step, in which the Cu(II) salt slurry is converted into Cu(I) salt and phenol, is carried out in a cascade of at least two reactors in order

to prevent slipthrough of the Cu(II) salt particles as much as possible. Preferably three, in particular four reactors are used. The step is effected in such a manner that 30-90%, preferably 60-90%, of the Cu(II) salt is converted into Cu(I).
This may take from 0.05 to 8 hours, depending on the temperature. Preferably the temperature is chosen so that a retention time of 0.1-3 hours is sufficient. A very high temperature in this 2nd step entails the risk of formation of metallic copper, which is undesirable.

In contrast to what is stated in NL-A-78.07199, it has proved to be essential in the second step of the process according to the invention for water to be present in the reduction step. Further it has appeared that it is of advantage to use a temperature of 210-270 ° C, preferably a temperature of 220-250 ° C.

The formation of the phenol according to the invention should therefore take place at a temperature of 210-270 ° C, prferably 220-250 ° C.

Both the reduction and the formation of phenol are effected in the absence of oxygen, in contrast to the process described in NL-A-70.00685, referred to in the foregoing. Owing to the absence of oxygen, reoxidation of the Cu-containing catalyst in this process step is avoided, so that no (adverse) reactions appeared to occur between the phenol or its intermediate products and any oxidized catalyst products. Such consecutive reactions appeared to give rise to the formation of selectivity-reducing by-products (such as tar).

It is advantageous to use such an amount of water in the second step of the process according to the invention as to achieve virtual equimolarity relative to the amount of Cu(II). As a result, the reaction product obtained upon completion of the hydrolysis is virtually free of water, which is of advantage in the further upgrading to pure phenol of the reaction product thus obtained. The amount of water as a rule is 0.5-5 wt.%, preferably 1-3 wt.%.

The pressure under which the second process step is performed is not critical, but the advantage of raising the pressure to above atmospheric level is that it has a favourable effect on the reaction kinetics and that the volatility of the reaction product is reduced. The pressure to be applied will generally be between 0.1 and 2.5 MPa; higher pressures, though allowable, do not yield substantial improvements of the process.

After the second step the reaction mixture is subjected to an upgrading operation to separate and recover the phenol obtained. This can be done in ways known by themselves, for instance by distillation. Certainly if hardly any water is left in the reaction mixture (in contrast to the process described in NL-B-90.684, which is also referred to as the "wet route"), there is no longer any need to use an auxiliary material (such as toluene, to break up the phenol-water azeotrope) in the distillation. The bottom flow of the distillation, notably containing non-converted arylcarboxylic acid and the Cu-containing catalyst, can be recycled to the oxidation step, optionally after a purification step.

The process according to the invention is suitable in particular for the preparation of unsubstituted phenol from unsubstituted benzoic acid. This phenol can be used for instance as starting material both for phenol-formaldehyde resins and for the preparation of caprolactam, starting material for nylon-6, or for the preparation of bisphenol-A.

The invention will now be elucidated in the following examples, which should not be construed as limiting the invention.

## Examples

### Description of the diagram

In the diagram, A is the oxidation reactor, B1 and B2 are the decomposition and hydrolysis reactors and C is the phenol separation column.
Copper (I) benzoate is supplied to the oxidation reactor (A) via line (1). Via (2) and (3) air is led in and discharged, causing the copper to be oxidized. The Cu(II) benzoate crystallizes out and is supplied to the decomposition section via (4) and (7). Flow (6) is the recirculation flow, by which 1-20% is recycled from (4) to reactor A. Fresh benzoic acid can be supplied via (5). Lines 4 through 7 are preferably kept at a temperature which is 1-20 ° C, preferably 1-10 ° C, higher than the temperature in A in order to prevent precipitation (scaling) of copper.

The decomposition reactors B1 and B2 are provided with a $CO_2$ offgas line (11) and a water supply line (8). Via (10) the decomposition/hydrolysis product goes to the phenol separation section. Phenol is separated via (12), water is removed via (13). Line 14 represents a reboiler.

### Example I

In a set-up as described above, an oxygen-containing gas mixture was led through reactor A at 180 °C, a copper concentration of 7.5 wt.% and a magnesium concentration of 0.5 wt.% in benzoic acid. The $O_2$ concentration varied between 1 and 15 vol.% and was so controlled that 0.05 wt.% copper remained present as Cu(I). The result in reactor A was a 35% slurry. Lines 4 through 7 had a temperature of 190 °C and the recirculation flow (6) was 2% of the flow in line (4). The temperature in the two decomposition/hydrolysis reactors B1 and B2 was 230 °C. The retention time was such that the reaction mixture in (10) was a homogeneous solution of mainly Cu(I) benzoate in benzoic acid and phenol. About five cycles were performed, in which no scaling (deposit of copper in the lines) or clogging occurred. The selectivity to phenol was 100%. If no recicrculation flow (6) was supplied to reactor A, acute clogging occurred in the gas inlet system. If the temperature in lines 4 through 7 was 180 °C instead of 190 °C, 13% scaling had occurred after five cycles (i.e. 13% of the copper had deposited in the lines).

Example II

This example was performed analogously to example I; the temperature in reactors A, B1 and B2 was 225 °C. A 7% slurry was formed in reactor A. The flow in recirculation line (6) was 10% of the flow in line (4). The temperature in the lines was 226 °C. The selectivity to phenol was 100% and no clogging, nor scaling occurred. If no recirculation flow was installed, the temperature in the oxydation section became unstable and the gas inlet tube was clogged after three cycles. Besides Cu(I) benzoate, carbonized products appeared to be present in the system. If lines 4 through 7 were kept at the same temperature as the reactors (225 °), 2% of the copper had deposited in the lines after five cycles.

## Claims

**1.** Process for the preparation of a phenol by an oxidative decarboxylation in the liquid phase of a corresponding arylcarboxylic acid in the presence of a Cu(I)-containing catalyst, characterized in that the following process steps are performed:

a) oxidation of the catalyst at a temperature of 120-260 °C, with the temperature, retention time, oxidation agent, phenol concentration and Cu(I) concentration being chosen so that virtually no tar is formed,

b) reaction of the oxidized catalyst of step a) and formation of phenol in the absence of oxygen, in the presence of water, at a temperature of 210-270 °C,

c) separation of phenol and recycling of the (reduced) catalyst to step a),

4-10 wt.% Cu being present in the reaction mixture, in step a) such a quantity of Cu(I) salt being converted to Cu(II) salt that the quantity of Cu(II) salt is higher than its solubility product so that in step a) a slurry is formed, nucleation agents for crystallization being added in step a), step b) being carried out in a cascade of at least two reactors, and the reduction of the catalyst being continued so far that a homogeneous solution is obtained.

**2.** Process according to claim 1, characterized in that the oxydation of the catalyst is carried out with an oxygen-containing gas.

**3.** Process according to any one of the claims 1-2, characterized in that part of the slurry is recycled from step b) to step a).

**4.** Process according to any one of the claims 1-3, characterized in that step a) is carried out at a temperature of 150-190 °C.

**5.** Process according to any one of the claims 1-3, characterized in that the oxydation in step a) is carried out at a temperature of 191-260 °C, with such a quantity of oxygen being supplied that at least 0.04 wt.% copper remains present as Cu(I) salt.

**6.** Process according to claim 5, characterized in that stap a) is carried out at a temperature between 210-250 °C.

**7.** Process according to any one of the claims 1-6, characterized in that step b) is carried out with a virtually equimolar quantity of water relative to the quantity of Cu(II).

**8.** Process according to any one of the claims 1-7, characterized in that step b) is carried out in a cascade of four reactors.

**9.** Process according to any one of the claims 1-8, characterized in that step b) is carried out at a temperature of 220-250 °C.

**10.** Process according to any one of the claims 1-9, characterized in that the arylcarboxylic acid used is unsubstituted benzoic acid.

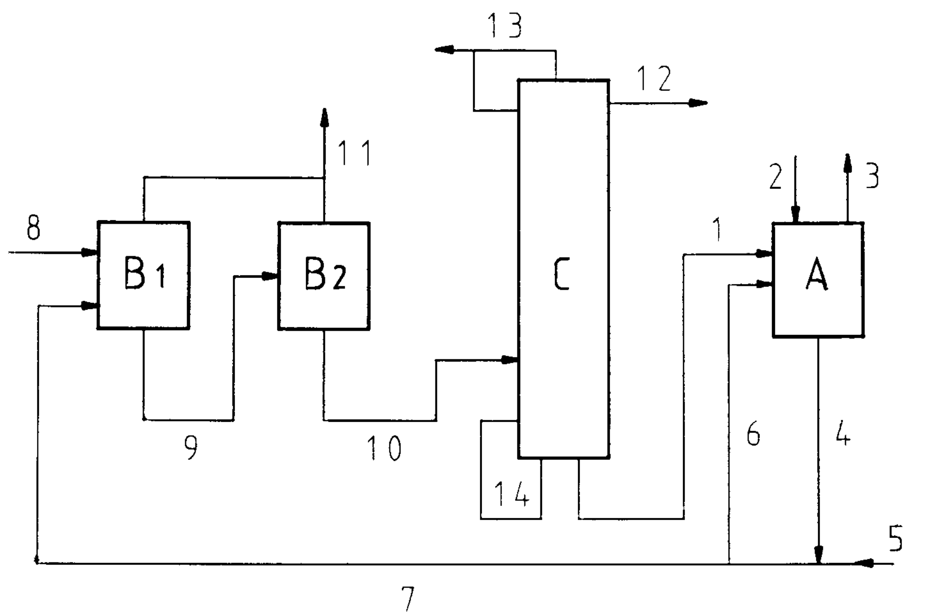
13
12
11
2
3
8
B1
B2
C
1
A
9
10
14
6
4
5
7

European Patent Office

# EUROPEAN SEARCH REPORT

Application Number

EP 92 20 1702

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E | EP-A-0 434 140 (STAMICARBON) * Whole document * --- | 1-9 | C 07 C 37/56<br>C 07 C 39/04 |
| X | US-A-4 405 823 (MAKI et al.) * Column 4, lines 17-48; column 5, lines 6-19; claims 1,5-7 * --- | 1,2,5,6,11,12 | |
| A | US-A-2 766 294 (W.G. TOLAND) * Column 2, lines 6-37; column 3, lines 25-35; claims 1-3 * --- | 1,2 | |
| A | FR-A-2 365 544 (STAMICARBON) * Page 1, line 29 - page 2, line 9 * ----- | 1-3 | |

TECHNICAL FIELDS SEARCHED (Int. Cl.5)

C 07 C 37/00
C 07 C 39/00

The present search report has been drawn up for all claims

| Place of search | Date of completion of the search | Examiner |
|---|---|---|
| THE HAGUE | 11-09-1992 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)